# EUROPÄISCHE PATENTANMELDUNG

(11) **EP 1 872 734 A1**
(43) Veröffentlichungstag der Anmeldung: **02.01.2008**
(21) Anmeldenummer: 06011590.4
(22) Anmeldetag: 06.06.2006
(51) Int. Cl.: A61B 18/20, A61C 1/00

(54) **Medizinischer oder kosmetischer Laser-Handgriff**

(71) Anmelder: W & H Dentalwerk Bürmoos GmbH, 5111 Bürmoos (AT)
(72) Erfinder: Strassl, Martin, 5020 Salzburg (AT); Irran, Thomas, 5020 Salzburg (AT)

(57) **Zusammenfassung**

Die vorliegende Erfindung betrifft einen medizinischen oder kosmetischen Handgriff (1) mit einer Lichtleitvorrichtung (5), die In einen ersten Abschnitt (5A) und in einen dazu gewinkelt angeordneten zweiten Abschnitt (5B) unterteilt ist, zur Abgabe von Laserstrahlung (10) auf eine Behandlungsstelle. Um die Laserstrahlung (10) möglichst effektiv vom ersten Abschnitt (5A) in den zweiten Abschnitt (5B) umzulenken und um insbesondere die Laserstrahlung (10) in einen die Laserstrahlung (10) weiterleitenden Lichtleiter (11B, 13B) möglichst verlustarm einzukoppeln, ist der Handgriff (1) mit einem zwischen den beiden Abschnitten (5A, 5B) angeordneten Reflektor (6) mit einer im Wesentlichen elliptisch ausgebildeten Reflexionsfläche (7) versehen.

## Beschreibung

Die vorliegende Erfindung betrifft einen medizinischen oder kosmetischen Handgriff zur Abgabe von Laserstrahlung auf eine Behandlungsstelle.

Ein derartiger Laser - Handgriff ist zum Beispiel aus der DE 37 135 12 A bekannt. Er umfasst einen Griffteil und einen gewinkelt dazu angeordneten Kopfteil mit der Lichtaustrittsöffnung. Zur Umlenkung der Laserstrahlung vom Griffteil in den Kopfteil und In die Lichtaustrittsöffnung ist ein als konkaver Spiegel ausgebildeter Reflektor vorgesehen. Alternativ sind zum Umlenken der Laserstrahlung auch andere optische Elemente wie Linsen, Planspiegel oder Prismen bekannt.

Allen bekannten optischen Umlenkelementen haften verschiedene Nachteile an, wie zum Beispiel keine oder eine nur ungenaue Fokussierung und ein damit verbundener Leistungsverlust, ein komplizierter Einbau in den Handgriff oder eine kostenintensive Herstellung.

Der vorliegenden Erfindung liegt daher die Aufgabe zugrunde einen Handgriff mit einem verbesserten optischen Element zur Umlenkung der Laserstrahlung zu schaffen. Das optische Element soll die Laserstrahlung möglichst effektiv umlenken und insbesondere einen möglichst großen Anteil der Laserstrahlung in einen die Laserstrahlung weiterleitenden Lichtleiter einkoppeln. Das verbesserte optische Element soll die gesamte Umlenkung somit möglichst verlustarm gestalten.

Diese Aufgabe wird gemäß der vorliegenden Erfindung durch einen Laser - Handgriff mit den Merkmalen des Anspruchs 1 gelöst. Besonders vorteilhafte Ausführungsformen sind in den Unteransprüchen angeführt.

Der erfindungsgemäße Laser - Handgriff weist zum Umlenken der Laserstrahlung vom ersten Abschnitt der Lichtleitvorrichtung in den gewinkelt dazu angeordneten zweiten Abschnitt einen Reflektor mit einer im Wesentlichen elliptisch ausgebildeten Reflexionsfläche auf. Die elliptische Reflexionsfläche hat insbesondere den Vorteil, dass im Vergleich zu allen bekannten optischen Elementen, die in medizinischen oder kosmetischen Laser - Handgriffen verwendet werden, eine deutlich verbesserte Fokussierung der Laserstrahlung erfolgt: Die im Wesentlichen durch den ersten Brennpunkt der elliptischen Reflexionsfläche geleitete Strahlung wird unabhängig von ihrem Austrittswinkel in den zweiten Brennpunkt der elliptische Reflexionsfläche umgelenkt, so dass die Umlenkung mit merklich geringeren Verlusten stattfindet als bei den bekannten optischen Element. Diese Wirkung ist bei den per Hand geführten medizinischen oder kosmetischen Laser - Handgriffen besonders wichtig, da die Handgriffe oftmals über Drehkupplungen, die dem Anwender eine freie Drehbarkeit und damit eine vereinfachte Handhabung ermöglichen, mit der Laserlichtquelle verbunden sind und die Laserstrahlung dem Handgriff mittels Lichtleiter zugeführt wird. Die Führung des Handgriffs per Hand und die Drehbarkeit haben zur Folge, dass der Lichtleiter oftmals ein geringes Bewegungsspiel in Bezug auf den Reflektor aufweist, so dass der Austrittswinkel der Strahlung in Richtung des Reflektors variiert. Ein Reflektor mit elliptischer Reflexionsfläche kann derartige Variationen besser ausgleichen als die bekannten optischen Umlenkelemente in medizinischen oder kosmetischen Handgriffen.

Der Reflektor mit der im Wesentlichen elliptischen Reflexionsfläche hat jedoch noch weitere Vorteile: So bewirkt er eine gleichzeitige Umlenkung und Fokussierung, so dass für beide Aufgaben nur ein optisches Element benötigt wird. Auch, wird dia sphärische Aberration gegenüber anderen bekannten optischen Umlenkelementen, die in medizinischen oder kosmetischen Laser - Handgriffen zum Einsatz kommen, verringert, was insbesondere für medizinische oder kosmetische Behandlungen wichtig ist, da hier die Strahlung nur auf die oftmals sehr kleine Behandlungsstelle auftreffen soll und das die Behandlungsstelle umgebende Gewebe möglichst frei von Bestrahlung bleiben soll. Schließlich eröffnet ein elliptischer Reflektor eine beträchtliche Freiheit in seiner Gestaltung (zum Beispiel in Bezug auf die Abmessung der beiden Achsen bzw. das Verhältnis der Achsenlängen zueinander, den Abstand der beiden Brennpunkte, den Krümmungsradius etc.), so dass der elliptischer Reflektor an die im Handgriff herrschenden räumlichen Gegebenheiten angepasst werden kann, ohne die oben genannten Vorteile, insbesondere die verbesserte Fokussierung, zu verlieren.

Der Reflektor mit der elliptischen Reflexionsfläche kann in einem ersten Ausführungsbeispiel als Hohlspiegel ausgebildet sein, Dies hat den Vorteil, dass der gesamte Reflektor ein geringes Gewicht aufweist, was insbesondere bei medizinischen oder kosmetischen Laser - Handgriffen, die vom Anwender lange in der Hand gehalten werden, eine wichtige Rolle spielt. In einem zweiten Ausführungsbeispiel ist der Reflektor als Vollkörper ausgebildet, d.h. als Lichtwellenleiter in Form eines für die Laserstrahlung transparenten Körpers mit einer elliptischen Reflexionsfläche. Der Vorteil des Vollkörpers liegt in den besseren Reflaxionseigonschaften (Totalreflexion) im Vergleich zum Hohlspiegel und in der einfacheren Fertigung, da die elliptische Reflexionsfläche als konvexe Außenfläche ausgebildet ist.

In einem zweiten Ausführungsbeispiel ist das dem Reflektor zugewandte Ende zumindest eines Abschnitts der Lichtleitvorrichtung im Wesentlichen in einem der Brennpunkte der elliptisch ausgebildeten Reflexionsfläche angeordnet In einem bevorzugten Ausführungsbeispiel ist das dem Reflektor zugewandte Ende zumindest eines Abschnitts der Lichtleitvorrichtung, insbesondere das Ende eines Glasstabs oder eines Glasfaserstabs, zwischen einem der Brennpunkte und der Reflexionsfläche angeordnet. Dies bedeutet, dass der Reflektor in Bezug auf die Lichtleitvorrichtung derart angeordnet, dass zumindest ein Brennpunkt der elliptisch ausgebildeten Reflexionsfläche in einen Abschnitt der Lichtleitvorrichtung verlegt ist, im Wesentlichen in jene Bereiche der Lichtleitvorrichtung, in denen die Spitze des Auskoppelkegels und / oder die Spitze des Akzeptanzkegels der Laserstrahlung ausgebildet wird. In vorteilhafter Weise erhält man dadurch eine weitere Steigerung der Lichtausbeute, da eine größerer Anteil der in der Lichtleitvorrichtung geleiteten Laserstrahlung vom Reflektor umgelenkt wird und auf dem zweiten Abschnitt der Lichtleitvorrichtung auftrifft.

In einem weiteren bevorzugten Ausführungsbeispiel entspricht die Geometrie der Im Wesentlichen elliptisch ausgebildete Reflexionsfläche einer Teilfläche der Oberfläche eines Ellipsoids, d.h. sie entspricht einem Ausschnitt der Oberfläche einer um ihre Achsen, insbesondere ihrer Hauptachse rotierenden Ellipse. Durch diese geometrische Ausformung wird erreicht, dass die Hauptintensität der umgelenkten Laserstrahlung in einer konzentrierten, näherungsweise kreis- oder punktförmigen Eintrittspupille auf dem zweiten Abschnitt der Lichtleitvorrichtung abgebildet wird, wobei die Fläche der Eintrittspupille bevorzugt geringer ist als die Eintrittsfläche des zweiten Abschnitts der Lichtleitvorrichtung, so dass eine möglichst verlustarme Einkopplung der Laserstrahlung erreicht wird.

Die Lichtleitvorrichtung bzw. jeder der beiden Abschnitte der Lichtleitvorrichtung besteht aus einer durch den Handgriff verlaufende Bohrung, die entweder als Strahlengang zur Leitung eines Freistrahls oder als Aufnahme für einen Glasstab oder eine optische Faser, insbesondere einen Glasfaserstab, ausgebildet sein kann. Der Glasstab oder die optische Faser können hierbei fix mit dem Handgriff verbunden sein und somit Teil des Handgriffs sein oder sie können lösbar im Handgriff aufnehmbar sein. Die höchste Wirkung in Bezug auf eine optimale und möglichst verlustfreie Fokussierung wird erreicht, wenn zur Leitung der Laserstrahlung ein Glasstab oder eine optische Faser verwendet werden. Der Winkel zwischen den Abschnitten der Lichtleitvorrichtung kann in einem weiten Bereich zwischen 5° - 90° liegen, in einer ersten Ausführungsform in einem Bereich von 5°- 40°, bevorzugt 15° - 20°, in einer anderen Ausführungsform in einem Bereich von 45° - 88°, bevorzugt 55° - 65° oder 70° - 75°.

Der Reflektor mit der elliptischen Reflexionsfläche kann einen metallischen, zum Beispiel aus Kupfer, Stahl oder Buntmetallen gefertigten, oder einen dielektrischen, zum Beispiel aus optischen Gläsern, insbesondere Quarzglas, Zink-Selenid-Glas oder Kalzium-Fluorid-Glas, Keramiken oder Kunststoffen hergestellten, Grundkörper umfassen. Der Reflektor kann des Weiteren eine metallische, zum Beispiel aus Kupfer, Stahl oder Buntmetallen gefertigte, oder eine dielektrische, bevorzugt aufgedampfte Beschichtung aufweisen. Zum Schutz vor Korrosion kann die Reflektoroberfläche zusätzlich eine Oxidschicht umfassen, zum Beispiel eine Silberoxid- oder Kupferoxidschicht. Bevorzugt können auch Mehrschichtsysteme, bestehend aus mehreren Lagen von Beschichtungen, auf dem Grundkörper aufgetragen werden, so dass zum Beispiel dichroitische oder so genannte gechirpte, d.h. die abweichenden Laufzeiten von mehreren Wellenlängen umfassenden Strahlungen korrigierende, Beschichtungen entstehen. Schließlich kann der Reflektor auf seiner Reflexionsfläche auch noch dreidimensionale Strukturen, zum Beispiel Rillen oder flächige Vertiefungen aufweisen, um über zusätzliche Reflexions-, Brechungs- oder Beugungseffekte dem reflektierten Strahl besonders vorteilhafte Formen und Intensitätsverteilungen aufzuprägen.

Die Kennwerte eines in einem erfindungsgemäßen medizinischen oder kosmetischen Laser - Handgriff aufgenommenen elliptischen Reflektors können in etwa betragen:
- Achsverhältnis der Haupt- zur Nebenachse: 1,25 - 6, bevorzugt 1,3 - 4, besonders bevorzugt 1,45.
- Brennweite: 1,5 - 5,9 mm, bevorzugt 1,6 - 4 mm, besonders bevorzugt 1,98 mm.
- Numerische Exzentrizität: 0,5 - 0,9, bevorzugt 0,6 - 0,8, besonders bevorzugt 0,725.

Die Erfindung wird nachfolgend anhand bevorzugter Ausführungsbeispiele und Bezug nehmend auf die belgefügten Zeichnungen erläutert:
Figur 1 zeigt ein Ausführungsbeispiel eines erfindungsgemäßen medizinischen oder kosmetischen Laser - Handgriffs.
Figur 2 zeigt eine schematische Darstellung eines in einem Laser - Handgriff enthaltenen Reflektors mit einer elliptischen Reflexionsfläche in Form eines Hohlspiegels.
Figur 3 zeigt eine schematische Darstellung eines in einem Laser - Handgriff enthaltenen Reflektors mit einer elliptischen Reflexionsfläche in Form eines Vollkörpers.

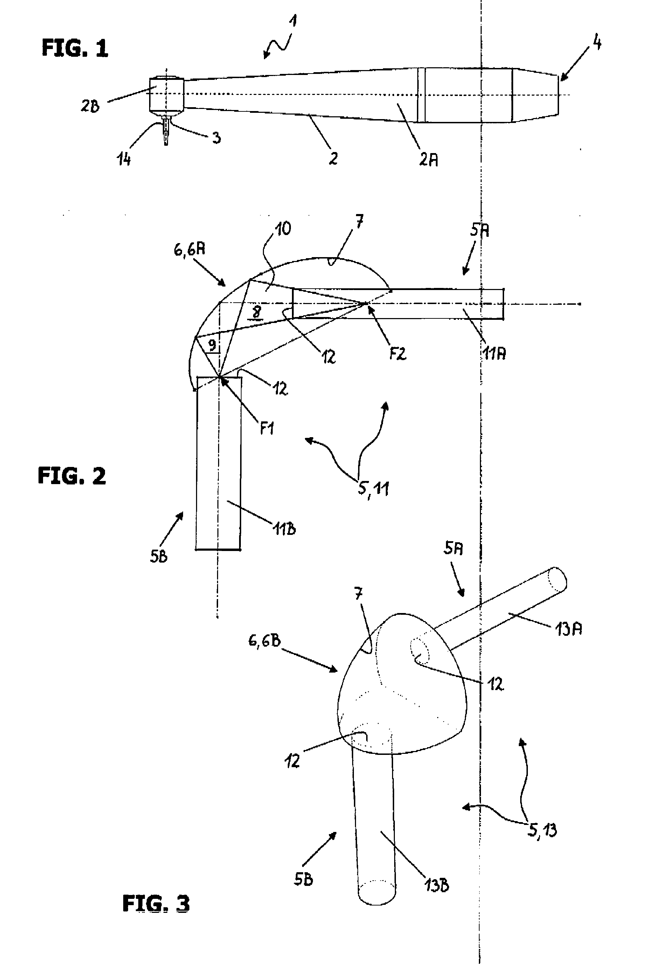

Der in Figur 1 dargestellte medizinische oder kosmetische Laser - Handgriff 1 ist Insbesondere für dentale Behandlungen einsetzbar. Er umfasst eine längliche, zylindrische, innen hohle Außenhülse 2, die in einen Griffteil 2A und einen Kopfteil 2B unterteilt ist. Griffteil 2A und Kopfteil 2B sind gewinkelt zueinander angeordnet, wobei der Winkel etwa 90° beträgt: Griffteil 2A und Kopfteil 2B können zweiteilig hergestellt und miteinander verbunden sein oder sie können aus einem Werkstück gefertigt sein. Am dem dem Kopftell 2B gegenüberliegenden Ende des Griffteils 2A ist eine Anschlussvorrichtung 4, zum Anschluss des Laser - Handgriffs 1 an eine Quelle für Laserstrahlung (nicht dargestellt) vorgesehen. Da die Anschlussvorrichtung bzw. die verschiedenen Arten von Anschlussvorrichtungen, die mit einem derartigen Handgriff 1 verwendbar sind, bekannt sind, wird darauf nicht im Detail eingegangen. Bevorzugt ist als Anschlussvorrichtung 4 eine Drehkupplung vorgesehen, die dem Anwender eine freie Drehbarkeit und damit gute Handhabbarkeit des Handgriffs 1 ermöglicht. Besonders bevorzugt sind die Drehkupplung und der Handgriff 1 derart ausgebildet, dass sie mit einem Versorgungsschlauch mit einer über den Versorgungsschlauch hinausragenden optischen Faser verbindbar sind. Die optische Faser wird dabei erst während des Kuppelns des Handgriffs 1 mit dem Versorgungsschlauch in den Handgriff 1 eingesteckt, wobei sie sich bevorzugt bis in die Nähe des Kopfteils 2B oder bis in den Kopfteil 2B erstreckt.

Am Vorderende der Außenhülse 2, insbesondere im Kopfteil 2B ist eine seitlich angeordnete Lichtaustrittsöffnung 3 vorgesehen, durch die Laserstrahlung 10 auf eine Behandlungsstelle abgegeben werden kann. Bevorzugt ist durch die Lichtaustrittsöffnung 3 ein Lichtleiter 14, zum Beispiel ein so genannter Saphir - Tip, in den Kopfteil 2B eingesteckt oder einsteckbar, welcher die Laserstrahlung 10 auf die Behandlungsstelle leitet. Das Vorderende des Lichtleiters 14 kann bevorzugt so ausgebildet sein, dass der Lichtleiter 14 auch direkt auf die Behandlungsstelle aufsetzbar ist, wodurch eine zielgenaue Applikation der Laserstrahlung und das Abstützen des Handgriffs 1 möglich sind.

Durch den Handgriff 1 von der Anschlussvorrichtung 4 zur Lichtaustrittsöffnung 3 erstreckt sich eine Lichtleitvorrichtung 5, die in einen ersten Abschnitt 5A und in einen dazu gewinkelt angeordneten zweiten Abschnitt 5B unterteilt ist. Die Lichtleitvorrichtung 5 umfasst eine erste und zweite Bohrung entsprechend den Abschnitten 5A, 5B und darin aufnehmbare optische Lichtleiter, zum Beispiel in Form eines optischen Glasfaserstabs 11, eines optischen Kristalls, insbesondere einem Saphirkristall, oder eines Glasstabs 13, die ebenfalls einen ersten Abschnitt 11A, 13A und einen dazu gewinkelt angeordneten zweiten Abschnitt 11B, 13B aufweisen (siehe Figuren 2 und 3). Die Abschnitt 5A, 11 A, 13A der Lichtleitvorrichtung 5 sind im Griffteil 2A und die Abschnitt 5B, 11B, 13B sind im Kopfteil 2B des Handgriffs 1 angeordnet. Abschnitt 11A ist bevorzugt das Endstück des in der Versorgungsleitung aufgenommenen Lichtleiters, über den der Handgriff 1 mit der Laserlichtquelle verbindbar ist. Das Endstück ragt über den Kupplungsabschnitt der Versorgungsleitung hinaus, so dass, wenn der Handgriff 1 mit der Versorgungsleitung verbunden wird, dieses Endstück in die Bohrung des Griffteils 2A einsteckbar ist und bis zum oder in den Kopfteil 2B des Handgriffs 1 ragt, Abschnitt 11 B der Lichtleitvorrichtung 5 kann bevorzugt als Teil des Lichtleiters 14 ausgebildet sein.

Figur 2 zeigt in schematischer Darstellung wie die Laserstrahlung 10 vom ersten Abschnitt 5A in den dazu gewinkelt angeordneten zweiten Abschnitt 5B der Lichtleitvorrichtung 5 übertragen wird. Zwischen den beiden Abschnitten 5A, 5B ist ein Reflektor 6 vorgesehen, dessen Reflexionsfläche 7 im Wesentlichen elliptisch ausgebildet ist, bevorzugt als Teilfläche eines Ellipsoids. Der Glasfaserstab 11A als Teil des ersten Abschnitts 5A leitet die Laserstrahlung 10 in Richtung des Reflektors 6 und strahlt sie an seinem dem Reflektor 6 zugewandten Ende 12 ab. Die abgestrahlte Laserstrahlung 10 trifft auf den als Hohlspiegel 6A ausgeführten Reflektor 6 und wird von diesem in den zweiten Abschnitt 5B der Lichtleitvorrichtung 5 mit dem Glasfaserstab 11B umgelenkt und in Richtung der Behandlungsstelle weitergeleitet. Die elliptische Reflexionsfläche 7 hat zwei Brennpunkte F1, F2, wobei jeder Strahl der Laserstrahlung 10, der von einem Brennpunkt F1 ausgeht, in den zweiten Brennpunkt F2 reflektiert wird.

Aus Figur 2 ist des Weiteren ersichtlich, dass die Abschnitte 5A, 11A bzw. 5B, 11B der Lichtleitvorrichtung 5 in Bezug auf den Reflektor 7 unterschiedlich angeordnet sein können: Das dem Reflektor 6 zugewandte Ende 12 des Glasfaserabschnitts 11A bzw. des Abschnitts 5A ist zwischen dem Brennpunkt F2 und der Reflexion fläche 7 angeordnet, Abschnitt 5A dringt somit in die Aushöhlung des Hohlspiegels 6A ein, Dadurch ist Brennpunkt F2 in die Abschnitte 5A, 11A der Lichtleitvorrichtung 5 verlegt, im Wesentlichen in jene Bereiche der Abschnitte 5A, 11A, in denen die Spitze des Auskoppelkegels 8 der Laserstrahlung 10 ausgebildet wird. Im Gegensatz dazu liegt das dem Reflektor 6 zugewandte Ende 12 des Abschnitts 5B der Lichtleitvorrichtung 5 im Wesentlichen im Brennpunkt F1 der elliptisch ausgebildeten Reflexionsfläche 7. Der Akzeptanzkegel 9 der Laserstrahlung 10 liegt hier dem gemäß außerhalb der Abschnitte 5B, 1B.

Die in Figur 2 dargestellte Ausführungsform stellt die wirksamste Anordnung der Abschnitte 5A, 5B, 11A, 11B dar, da durch das Einrücken der Abschnitte 5A, 11A in den Hohlspiegel 6A und durch das Positionieren der Abschnitte 5B, 11B im Wesentlichen im Brennpunkt F1 eine besonders wirkungsvolle Umlenkung erzielt wird, d.h. eine besonders große Menge an Laserstrahlung 10 so in die Abschnitte 5B, 11B eingekoppelt wird, dass diese auch weitergeleitet werden kann. Dennoch ist die dargestellte Ausführungsform nur exemplarisch und selbstverständlich können, wenn technisch notwendig oder zweckmäßig, zum Beispiel bei beengten Platzverhältnissen, auch die Abschnitte 5B, 11 B in den Hohlspiegel 6A eingeschoben sein und / oder das Ende 12 der Abschnitte 5A, 11A im Wesentlichen im Brennpunkt F2 liegen.

Das in Figur 3 dargestellte Ausführungsbeispiel entspricht in Funktion und grundsätzlichem Aufbau jenem aus Figur 2, so dass gleiche Bauteile mit denselben Bezugszeichen versehen sind. Im Unterschied zu Figur 2 ist der Reflektor 6 als Vollkörper 6B ausgestaltet, mit einem zum Beispiel aus optischem Glas, wie Quarzglas, bestehendem Grundkörper, wobei eine Seitenwand dieses Grundkörpers als im Wesentlichen elliptisch ausgebildete Reflexionsfläche 7, bevorzugt als Teilfläche der Oberfläche eines Ellipsoids geformt ist. Der Reflektor 6 ist somit selbst ein Lichtleiter, wobei die Laserstrahlung durch den einen Glasstab 13A umfassenden Abschnitt 5A zum Vollkörper 6B und durch diesen bis zur Reflexionsfläche 7 geleitet wird. Nach der Umlenkung an der Reflexionsfläche 7 tritt die Laserstrahlung in den Glasstab 13B des Abschnitts 5B ein und wird in Richtung der Behandlungsstelle weitergeleitet. Wie für Figur 2 beschrieben können ein oder beide Enden 12 der Glasstäbe 13A, 13B wiederum im Wesentlichen im Brennpunkt F1, F2 der Reflexionsfläche 7 oder zwischen dem Brennpunkt F1. F2 und der Reflexionsfläche 7 angeordnet sein. Weiters besteht die Möglichkeit zumindest eines der Enden 12 in einer Sacklochbohrung des Vollkörpers 6A aufzunehmen, wodurch der Zusammenbau der Lichtleitvorrichtung 5 mit dem Reflektor 6 vereinfacht und die Positionierung der Lichtleitvorrichtung 5 relativ zur Reflexionsfläche 7 stabilisiert wird.

Die Abmessungen der in den Figuren 2 und 3 dargestellten Reflektoren 6 betragen in etwa 3,0 mm/7,0 mm 13,5 mm (Länge/ Breite/ Höhe).

Die Erfindung ist nicht auf die beschriebenen Ausführungsbeispiele beschränkt, sondern umfasst alle Ausführungsmöglichkeiten, die das prinzipielle, sinngemäße Funktionsprinzip der Erfindung nicht verändern. Insbesondere sind derartige Handgriffe nicht nur für dentale, sondern auch für viele andere kosmetische oder medizinische Behandlungen einsetzbar, zum Beispiel für ophthalmologische Eingriffen, für die Entfernung von Haaren oder von Muttermalen, in der Gefäßmedizin oder für diagnostische Anwendungen.

## Patentansprüche

1. Medizinischer oder kosmetischer Laser - Handgriff (1) mit einer Außenhülse (2), einer Lichtaustrittsöffnung (3) zur Abgabe von Laserstrahlung (10) auf eine Behandlungsstelle, einer Anschlussvorrichtung (4) zum Anschluss des Laser - Handgriffs (1) an eine Quelle für Laserstrahlung, einer sich von der Anschlussvorrichtung (4) zur Lichtaustrittsöffnung (3) erstreckenden und zumindest eine Bohrung umfassenden Lichtleitvorrichtung (5), wobei die Lichtleitvorrichtung (5) einen ersten Abschnitt (5A) und einen dazu gewinkelt angeordneten zweiten Abschnitt (5B) aufweist, und mit einem zwischen den beiden Abschnitten (5A, 5B) angeordneten Reflektor (6) mit einer im Wesentlichen elliptisch ausgebildeten Reflexionsfläche (7) zur Umlenkung der Laserstrahlung (10) vom ersten Abschnitt (5A) in den zweiten Abschnitt (5B) der Lichtleitvorrichtung (5).

2. Laser - Handgriff (1) nach Anspruch 1, **dadurch gekennzeichnet dass** die Reflexionsfläche (7) als Ausschnitt der Oberfläche eines Ellipsoids ausgebildet ist.

3. Laser - Handgriff (1) nach Anspruch 1 oder 2, **dadurch gekennzeichnet, dass** der Reflektor (6) als Hohlspiegel (6A) oder als Vollkörper (6B) ausgebildet ist.

4. Laser - Handgriff (1) nach einem der Ansprüche 1-3, **dadurch gekennzeichnet, dass** das dem Reflektor (6) zugewandte Ende (12) zumindest eines Abschnitts (5A, 5B) der Lichtleitvorrichtung (5) zwischen einem der Brennpunkte (F1, F2) der Reflexionsfläche (7) und der Reflexionsfläche (7) angeordnet ist.

5. Laser - Handgriff (1) nach einem der Ansprüche 1-3, **dadurch gekennzeichnet, dass** das dem Reflektor (6) zugewandte Ende (12) zumindest eines Abschnitts (5A, 5B) der Lichtleitvorrichtung (5) Im Wesentlichen in einem der Brennpunkte (F1, F2) der elliptisch ausgebildeten Reflexionsfläche (7) angeordnet ist.

6. Laser - Handgriff (1) nach einem der vorhergehenden Ansprüche, **dadurch gekennzeichnet, dass**
der Reflektor (6) einen metallischen oder dielektrischen Grundkörper umfasst.

7. Laser - Handgriff (1) nach einem der vorhergehenden Ansprüche, **dadurch gekennzeichnet, dass**
die elliptische Reflexionsfläche (7) eine metallische oder dielektrische Beschichtung und / oder eine Oxidschicht umfasst.

8. Laser - Handgriff (1) nach einem der vorhergehenden Ansprüche, **dadurch gekennzeichnet, dass**
die Lichtleitvorrichtung (5) des Weiteren zumindest einen Glasstab (13A, 13B), einen optischen Kristall oder eine optische Faser, insbesondere einen Glasfaserstab (11A, 11B) umfasst.

9. Laser - Handgriff (1) nach einem der vorhergehenden Ansprüche, **dadurch gekennzeichnet, dass**
als Anschlussvorrichtung (4) eine Drehkupplung vorgesehen ist und dass die Drehkupplung und der Handgriff (1) derart ausgebildet sind dass sie mit einem Versorgungsschlauch mit einer über den Versorgungsschlauch hinausragenden optischen Faser verbindbar sind.
